# EUROPEAN PATENT SPECIFICATION

(11) **EP 3 858 848 B1**
(45) Date of publication and mention of the grant of the patent: **01.11.2023**
(21) Application number: 20878033.8
(22) Date of filing: 17.11.2020
(51) Int. Cl.: C07J 53/00, C07B 63/00

(54) **METHOD FOR PREPARING ORYZANOL BY USING SOAPSTOCK CONTAINING ORYZANOL AS RAW MATERIAL**
VERFAHREN ZUR HERSTELLUNG VON ORYZANOL AUS EINEM ALS ROHSTOFF DIENENDEN ORYZANOLHALTIGEN SEIFENSTOCK
PROCÉDÉ DE PRÉPARATION D'ORYZANOL À L'AIDE D'UNE PÂTE DE NEUTRALISATION CONTENANT DE L'ORYZANOL EN TANT QUE MATIÈRE PREMIÈRE

(30) Priority: 22.11.2019 CN 201911156181
(43) Date of publication of application: 04.08.2021
(73) Proprietor: Hunan Huacheng Biotech, Inc, Changsha City, Hunan 410205 (CN)
(72) Inventor: HUANG, Huaxue, Changsha City Hunan 410205 (CN); LIU, Genggui, Changsha City Hunan 410205 (CN); HE, Anle, Changsha City Hunan 410205 (CN); ZENG, Runqing, Changsha City Hunan 410205 (CN)
(74) Representative: Murgitroyd & Company
(86) International application number: PCT/CN2020/129370
(87) International publication number: WO 2021/098680

(56) References cited:
- WO-A1-2004/055040
- CN-A- 101 440 115
- CN-A- 102 464 696
- CN-A- 103 435 677
- CN-A- 110 294 784
- CN-A- 110 305 737
- CN-A- 110 746 478
- JP-A- 2015 224 237
- US-A1- 2004 192 948
- Zhou Diping, Tang Ke-Jun, Liu Geng-Gui, Jiang Xin-Fang, Luo Xi: "The New Technology of Separation and Extraction Content 98% Natural Ferulic Acid from the Waste of Rice Bran Oil Processing", Enterprise Science and Technology & Development, vol. 5, no. 323, 1 January 2012 (2012-01-01), pages 10-16, XP055813975, ISSN: 1674-0688
- Sereewatthanawut, I. ; Baptista, I.I.R. ; Boam, A.T. ; Hodgson, A. ; Livingston, A.G.: "Nanofiltration process for the nutritional enrichment and refining of rice bran oil", Journal of Food Engineering, Barking Essex, GB, vol. 102, no. 1, 1 January 2011 (2011-01-01), pages 16-24, XP027284600, GB ISSN: 0260-8774

## Description

### TECHNICAL FIELD

The present invention belongs to the technical field of plant extraction and separation, and relates to a method for preparing oryzanol and specifically to a method for preparing oryzanol from oryzanol-containing soapstock serving as a raw material.

### BACKGROUND

Oryzanol exists in rice bran oil and is a mixture of ferulic acid esters with triterpene (en) alcohol serving as a main body. It mainly acts on the autonomic nervous system and endocrine centre of the diencephalon, can adjust the autonomic nerve function, reduce endocrine balance disorders, and improve psychoneural disorders, and also has a variety of physiological functions such as lowering blood lipids, lowering liver lipids, preventing lipid oxidation, and resisting oxidation. In addition, people have recently used oryzanol as a raw material for skin care products and food antioxidants. Oryzanol-rich rice bran oil and other soapstock are generally used as raw materials to extract and prepare oryzanol, but products generally have unsatisfactory purity and yield.

There are also many domestic reports on oryzanol patented technology, but each has some shortcomings. The classification is detailed as follows.

### 1. According to solvents used, they are as follows:

Patents such as CN201710073967, CN201510769436, CN201310659297, CN201310099264, CN201010542976, CN201010224081, CN200810231528, and CN02135610 all use methanol as an extraction solvent, and then obtain oryzanol via a series of refining. Methanol is easy to remain, and this product does not meet export product regulations.

Patents such as CN201510769436 and CN201010542976 use hexane for refining or crystalisation, and then obtain oryzanol products via some processing steps.

JP Patent Application No. P2015-224237A discloses a method to prepare oryzanol from an oryzanol containing soapstock (rice bran) wherein:- rice bran in ethanol/water is saponified with a potassium hydroxide aqueous solution before adding a solvent mixture hexane / ethyl acetate 8:2 to the reaction mixture and its treatment with ultrasonic waves at 80°C; - the mixture is transferred to a centrifuge tube and centrifuged to separate the liquid phase and the solid content. A mixed solution of hexane / ethyl acetate (8:2) is added to the solid phase component, irradiation with ultrasonic waves is performed under the same conditions as above, before separating the liquid phase and solid component. The operation is repeated three times. All the obtained liquid phases are transferred to a separating funnel and separated. The pH is adjusted to 3-4 by adding 2M H₂SO₄ to the aqueous phase. - the organic phase obtained by the liquid separation is washed with water to adjust its pH to around 7, and then dried over anhydrous sodium sulfate. Sodium sulfate is filtered off and the solvent removed.

However, the hexane-related processes are prone to residual hexane, resulting in excess residues.

In the prior art, methanol is mostly used. This is mainly because methanol has high extraction efficiency for oryzanol, but needs to have high post-treatment requirement, otherwise, remaining methanol will exceed the standard. Moreover, the large-scale use of methanol in the production process is also potentially dangerous to the health of operators.

### 2. According to processes, they are as follows:

The patent application with application number CN201710073967.6 discloses an oryzanol extraction process: first degumming crude rice oil to obtain degummed oil; performing alkali refining on the degummed oil to obtain soapstock; after soapstock is subjected to supplementary saponification, adding methanol, water and sodium carbonate for alkali dissolving to obtain the supernatant; adding hydrochloric acid and citric acid into the supernatant in sequence to adjust the pH to obtain a crude oryzanol product; and washing and drying the crude product to obtain refined oryzanol. This method uses alkaline methanol for extraction. The solvent is toxic and remains in the product. The sodium carbonate cannot be combined with phenolic hydroxyl groups in oryzanol molecules in practice due to its alkalescence, and cannot promote the dissolution of oryzanol.

The patent application with the application number CN201510769436.1 discloses a γ-oryzanol production method, comprising: pouring filter residues into a dissolving tank; adding methanol for mixing, and stirring with hexane; adding acetic acid for neutralisation, carrying out stirring and refluxing; heating the mixture in a water bath to 50°C, and carrying out stirring and refluxing at higher temperature; adding water to continue to stir the mixture, and standing for one hour; precipitating a crude product γ-oryzanol, wherein the solution is automatically layered, the upper layer being a hexane solution, and the lower layer being the crude product γ-oryzanol precipitates and a methanol dissolved layer; shunting the upper layer and the methanol dissolved layer on the lower layer; heating the lower layer in a water bath; carrying out vacuum filtration on the crude product γ-oryzanol precipitates and the methanol dissolved layer subjected to water-bath heating, wherein the upper layer of filter paper is crude γ-oryzanol; adding hexane into the crude γ-oryzanol precipitated particles, and washing out the impurities that can be dissolved in hexane; and collecting the γ-oryzanol precipitated particles, and drying the particles at a low temperature. Under a heating condition, oryzanol is easily dissolved in hexane, and has greater solubility in methanol. Methanol is more toxic and easily remains in the product. Since the oryzanol is dissolved in both of the solvents, the method cannot achieve the purpose of separation, and a further research is needed. Hexane is extremely flammable and explosive, so that the production safety needs to be further improved.

The patent with the application number CN201310659297.8 discloses a method for extracting oryzanol from waste bran meal oil, comprising the following steps: 1) adding high temperature resistant enzyme into the waste bran meal oil at an addition amount of 0.5-5% of the mass of the waste bran meal oil, stirring and digesting them at 70-95°C for 60-120min, and filtering the mixture to obtain grease clear liquid; 2) performing alkali dissolving: mixing an alkaline methanol solution with the grease clear liquid of the step 1) in a mass ratio of 1:5-1:8, heating to 60°C, stirring the materials, standing until the two phases are separated, and carrying out extraction; 3) performing acid precipitation: heating the extract of the step 2) to 40-60°C, adding acid to adjust the pH to 3.0-6.0, standing it overnight, and carrying out centrifugal separation to obtain a crude oryzanol product; 4) refining oryzanol: flushing the crude oryzanol product obtained in the step 3) with distilled water, and then drying the product to obtain refined oryzanol. The chemical nature of enzymes is protein, and most enzymes will be inactivated above 70°C. The scientific nature of the method needs to be studied. Methanol is a toxic reagent, and oryzanol is a water-insoluble substance, impurities from which are also derived from oil and are also strong lipophilic substances. It is impossible to achieve the purpose of refining after the product is simply flushed with the distilled water and dried. The refining method needs to be further studied.

The patent with the application number CN201310099264.2 discloses a method for extracting oryzanol from crude rice bran oil, comprising the following steps: (1) preparing four-level rice bran oil: a, hydrating the crude rice bran oil for degumming and dewaxing; b, decolourising the dewaxed rice bran oil; c, physically refining the decolourised rice bran oil ; (2) extracting crude oryzanol: a, preparing a soap base; b, carrying out an alkali dissolving operation; c, carrying out an acid precipitation operation; and (3) refining crude oryzanol: a, redissolving the crude oryzanol, and carrying out liquid washing with solvent oil; b, carrying out acid precipitation and acid soaking; c, drying and smashing oryzanol. This method uses toxic methanol which would remain in the product; there is no crystallisation step for the product, so that the content needs to be further confirmed; and the solvent oil is extremely flammable and explosive, so that the production safety needs to be further improved.

The patent with the application number CN201010542976.3 discloses a process for extracting oryzanol from rice bran, comprising: adding methanol into crude bran oil, adjusting the pH with sodium hydroxide, heating the mixture, stirring the mixture under a heat preservation condition, cooling the mixture, and standing for layering, and discharging a lower oil phase; adding citric acid into a methanol solution at the upper layer to adjust the pH to 7, standing for filtering and precipitation to obtain a crude product; and recrystallising the crude product with hexane, and washing and drying the product to obtain a refined product. This method uses toxic methanol which would remain in the product. Hexane is extremely flammable and explosive, so that the production safety needs to be further improved.

The patent with application number CN201010224081.5 discloses a method for extracting oryzanol by non-polar solvent extraction. Rice bran oil base is used to refine soapstock; a methanol ether alkaline solution is added, and the mixture is fully stirred and then stands for layering, the upper layer being a neutral substance and the lower layer being an oryzanol-containing alkaline substance; a hydrochloric acid solution is used to adjust the pH value; and at this time, there are oryzanol crystals precipitated. Then, the oryzanol crystals are washed and vacuum-dried to obtain a finished oryzanol product. This method uses toxic methanol and ether, and there will be methanol remaining in the product; ether is a controlled reagent, and is extremely flammable and explosive, so that the production safety needs to be further improved; and whether the content of oryzanol precipitated by acid adjustment meets the standard needs to be further confirmed.

The patent with application number CN200810231528.8 discloses a production method for extracting oryzanol from rice bran oil soapstock. Oryzanol-rich rice bran oil soapstock is diluted with methanol after being heated and dissolved, and then is added with alkali for supplementary saponification; later, alkali dissolution and filtration are carried out to obtain filtrate of saponified liquid; the filtrate of the saponified liquid is then filtered; and after water washing, acid leaching, purification and granulation are carried out, and then the product is dried. This method uses toxic methanol which would remain in the product; the main process is alkali-alcohol dissolution and acid precipitation; and after simple purification, whether the content of oryzanol meets the standard needs to be further confirmed.

The patent with application number CN200810244052.8 discloses an extraction method for oryzanol. Crude rice bran oil is used as a raw material. Firstly, ethanolamine is used to pre-deacidify mixed oil of rice bran oil and n-hexane, and then a NaOH solution is used to enrich oryzanol into soapstock; methanol is used to extract oryzanol sodium salt in the soapstock; and finally, an acidic solution is used to adjust the pH value to precipitate oryzanol crystals, and oryzanol is obtained after separation. This method uses toxic methanol which would remain in the product; the main process is alkali-alcohol dissolution and acid precipitation; and the obtained product is low-content oryzanol.

The patent with application number CN02135610.6 discloses a method for extracting oryzanol by solvent extraction. Firstly, crude rice bran oil is pretreated, and the pretreated bran oil is physically refined to remove free fatty acids from the oil; alkaline methanol is added into the refined bran oil for continuous countercurrent extraction; acid is added into the extracted methanol mother liquor to precipitate the oryzanol crystals contained therein; and the oryzanol is obtained after separation. This method uses toxic methanol which would remain in the product; the main process is alkali-alcohol dissolution and acid precipitation; and the obtained product is low-content oryzanol.

It can be seen that there are three main problems in oryzanol currently on the market: 1. Excessive solvent residues and the use of illegal solvents: This is mainly due to the organic solvents such as methanol, ether and hexane and organic solvent remainings, resulting in that oryzanol products cannot be exported to Europe, America and Japan. The green export standard stipulates that export products shall not use toxic solvents such as methanol in a production process. 2. The actual contents of most oryzanol products are not up to the standard: Although some commercial products are white, the content is only about 95%. The analysis shows that these products produce some new degradable substances, which are also white, in the production process, but the content of oryzanol is reduced, which is related to a production technology. 3. The colours of some oryzanol products do not meet the standard: Although the content of the product reaches 98%, there is often no decolourisation step, so that the colour of such a product is off-white or grey white, and yellowish white, and sales of the product to Europe and America is restricted. Therefore, there is an urgent need to propose an oryzanol production technology, so that the product yield and purity can reach a satisfactory level, and the production technology is environmentally friendly, and does not use toxic or flammable or explosive reagents.

### SUMMARY

The technical problem to be solved by the present invention is to overcome the above-mentioned shortcomings in the prior art and provide a preparation method for oryzanol. Solvents used in a production process and solvent residues meet an export standard; the production safety is improved; highly toxic substances such as methanol, and extremely flammable and explosive organic solvents such as ether, hexane and solvent oil are not used; a production technology suitable for industrialisation is built to produce high-quality oryzanol industrial products on a large scale; the actual content of oryzanol in a product is greater than 98%, and the yield is greater than 73%; white industrial products are excellent in purity and colour performance; the production cost is reduced; and the economical and social benefits of an enterprise are increased.

The technical solution used by the present invention to solve the technical problems is as follows: A method for preparing oryzanol from oryzanol-containing soapstock serving as a raw material comprises the following steps:
1), alkali-alcohol thermal dissolution: adding ethanol into the raw material, heating to 50-60°C, adjusting the system to alkalescence, and dissolving the raw material to obtain a solution;
2), heat-preserved fine filtration: maintaining the temperature in the step 1), and using a precision filter to filter the solution;
3), heat-preserved ultrafiltration membrane separation: under a condition of 50-60°C, carrying out membrane separation on the filtrate by a high temperature resistant ultrafiltration membrane, and collecting membrane separation liquid in a molecular weight range cut-off of 1000-5000;
4), heat-preserved decolourisation: adding an inorganic decolouriser into the membrane separation liquid, and fully stirring and filtering the liquid to obtain decolourised liquid;
5), acid neutralisation and filtration: neutralising the decolourised liquid with dilute acid, cooling the liquid, standing the liquid to completely separate out precipitates, carrying out centrifugation, and collecting the centrifugated precipitates;
6) washing: adding ethyl acetate, ethanol at a concentration greater than 85% and purified water with a temperature of 40-45°C in sequence into the centrifugated precipitates for washing, wherein the washing is to add a washing liquid of the above solvent or solution into the centrifugated precipitates, stir and mix the product thoroughly, and centrifugate the mixture to obtain centrifugated precipitates for next step of washing; and
7) drying: drying the precipitates to obtain a finished oryzanol product, wherein in the step 1) to the step 4) the system is maintained at a temperature of 50-60°C; and wherein in the step 4), the decolouriser comprises activated clay and activated carbon at a mass ratio of 1-2:1-2, and the adding amount of the decolouriser is 3-6% by weight of the oryzanol-containing soapstock raw material.

Preferably, at the steps 1) to 4) of the above method of the present invention, the temperature of the system is maintained at 50-60°C. The temperature is controlled within the above range, and cooperates with process conditions and parameters of other steps, so that the efficiency of extracting the oryzanol can be improved to the maximum extent, and the purity of the oryzanol in the product can be guaranteed, thereby meeting the requirements of industrial production and preparation of high-quality oryzanol-containing products.

Preferably, at the step 1), the ethanol refers to ethanol at a concentration greater than 80%, preferably ethanol at a concentration greater than 85%. More preferably, ethanol at a concentration greater than 85% is used; the adding amount of the ethanol is 5-10 times the weight of an oryzanol-containing soap base. The heating refers to heating till the temperature is 50-60°C; the adjustment to alkalescence refers to use of an alkaline hydroxide, such as sodium hydroxide, and the hydroxide to adjust the pH to 8.5-9.5.

It was reported in the literature that oryzanol was extracted by adjusting the pH with methanol before. The inventor found that using high-concentration edible ethanol and controlling the temperature at 50-60°C throughout the process can also extract oryzanol, and the oryzanol meets the solvent usage and residue standard for export products. Technically speaking, the oryzanol extracted from ethanol is light in colour, contains fewer impurities, and is high in purity. If the concentration of the edible ethanol is low, most of the oryzanol cannot be extracted, leading to large loss; if the concentration of the edible ethanol is high, it is difficult to recycle the high-concentration edible ethanol during production. The heating temperature is 50-60°C. If it is high or the dissolution time is long, the heating temperature will have a significant damaging effect on oryzanol under an alkaline condition and cause a serious decrease in product yield; if it is low or the dissolution time is short, the heating temperature will not be conducive to the dissolution of oryzanol, and insoluble matters will increase, so that the product yield is reduced, and the filtration rate of fine filtration is low; the finally adjusted pH of the solution which is too large will significantly destroy oryzanol, resulting in low oryzanol yield; a too small pH is not conducive to the dissolution of oryzanol, resulting in low yield. In a suitable pH range, the system has no influence on the membrane in the subsequent membrane separation operation, and is within the pH range that the membrane can withstand.

Preferably, at the step 2), the filtration temperature is controlled at 50°C-60°C; for filtration, a precision filter with a folding filter element is used. The filter element has a pore size of 0.45 µm and an operating pressure of 0.2-0.4 MPa.

If it is greater than 60°C, the temperature will have a significant destroying effect on oryzanol under the alkaline condition and cause a serious decrease in product yield; and if the temperature is less than 50°C, the dissolution of oryzanol will be reduced, and the insoluble matters will increase, so that the product yield is reduced, and the filtration rate of fine filtration is greatly reduced, thereby leading to low production efficiency and easy blockage of the filter element. In addition, the inventors find through many experiments that the above-mentioned folding filter element selected has a good filtering effect on the oryzanol-containing highly ethanolic solution, and the obtained filtrate is a clear and transparent solution; and under the same conditions, filtration materials such as microporous membranes are easily blocked and cannot be operated normally. Selecting a proper pore size for the filter element is to match the clarity requirement of the ultrafiltration membrane separation for the solution. If the pore size is too large, more impurities will pass through, which will affect the service life of the ultrafiltration membrane; and a small pore size will slow down the filtration and affect the production efficiency. The operating pressure of the precision filter is affected by the filter element. When impurities accumulated on the surface of the filter element increase, the corresponding operating pressure increases. When the pressure increases to a certain level, the filter element needs to be cleaned in time to ensure the filtering effect and rate.

Preferably, at the step 3), ultrafiltration is carried out at 50-60°C, and the high temperature resistant ultrafiltration membrane used is made of polyethersulfone, such as the Duratherm series of GE (specific ions comprise NF 8040HF, NF RO8040, RO 8040HR, NF 8040HR, NF 3840HR, FLD-UF). It can withstand a temperature of 70°C. At the step 2), the heat preservation temperature of the filtrate is 50°C-60°C, which is the same as the heat preservation temperature of the ultrafiltration membrane separation operation. An ordinary ultrafiltration membrane can only withstand a temperature within 45°C. Therefore, the present invention shows through lots of investigations that the high temperature resistant ultrafiltration membrane can withstand a temperature of 70°C, adapting to the heat-preserved membrane separation condition of the oryzanol solution. The temperature during ultrafiltration is set at 50-60°C. If it is greater than 60°C, the temperature will have a significant destroying effect on oryzanol under the alkaline condition and cause a serious decrease in product yield; and if the temperature is less than 50°C, the dissolution of oryzanol will be reduced, and the insoluble matters will increase, so that the product yield is reduced, and the operation rate of ultrafiltration membrane is greatly reduced, thereby leading to low production efficiency and easy blockage of the ultrafiltration membrane.

More preferably, at the step 3), the molecular weight cut-off of the membrane is set to be 1000-5000, and a membrane operating pressure is 1.0-2.0 MPa. After it is determined that the type and performance of the membrane meet the requirements, it is necessary to further select the molecular weight cut-off of the ultrafiltration membrane according to the molecular weight and chemical structure of oryzanol, and the types of impurities. The present invention shows through a large number of experiments that 95% of the oryzanol in the solution is distributed in a range 1000-5000 of the molecular weight cut-off range of the membrane, and the colour of the solution turns from reddish brown to yellow, that is, significant decolourisation and purification effects are achieved by means of the membrane separation with this molecular weight. The ultrafiltration membrane needs to be operated at a proper pressure. An extremely low pressure will reduce the membrane filtration rate, and a high pressure will easily cause loss of oryzanol.

Further preferably, at the step 3), the order of the membrane separation is: firstly, the solution is filtered with an ultrafiltration membrane with a molecular weight cut-off of 5000; membrane downstream liquid, i.e., permeated liquid, is then collected; the permeated liquid is filtered with an ultrafiltration membrane with a molecular weight cut-off of 1000; and membrane upstream liquid, i.e., cut-off liquid, is collected. The ultrafiltration is carried out in the above order, which can further improve the separation and purification effects. At the step 4), the temperature is still maintained at 50-60°C during decolourisation. If it is greater than 60°C, the temperature will have a significant destroying effect on oryzanol under the alkaline condition and cause a serious decrease in product yield; and if the temperature is less than 50°C, the dissolution of oryzanol will be reduced, and oryzanol adsorbed by the inorganic decolouriser increases, so that the product yield is reduced, and plate and frame filtration is difficult.

Preferably, at the step 4), the decolouriser is at least one of activated clay and activated carbon, and the adding amount of the decolouriser is 3-6% of the weight of the oryzanol-containing soapstock raw material. More preferably, the decolouriser is a compound of activated clay and activated carbon according to a mass ratio of 1-2:1-2. The present invention proves through experiments that the activated clay has good degreasing and decolourisation effects on fat-soluble components with grease, and can assist filtration; and the activated carbon also has a good decolourisation effect in polar solutions and can assist filtration. The activated clay and the activated carbon work collaboratively to have better degreasing and decolourisation effects, and have the least impact on the final oryzanol yield.

Further preferably, at the step 4), the filtration manner is plate and frame filtration taking canvas filter cloth as a filtration material. The plate and frame filtration can completely remove tiny activated clay and activated carbon particles fast. Therefore, under a heat preservation condition, a light yellow, clear and transparent decolourised liquid can be obtained through the plate and frame filtration. Other filtration manners can be used, but they cannot effectively remove smaller-granularity inorganic decolourisers, or have long filtration time. A long-time filtration manner is not conductive to reducing the energy consumption and reducing the cost since the temperature in the whole process needs to be maintained at 50-60°C in the present invention.

At the step 5), the dilute acid is dilute hydrochloric acid or dilute sulfuric acid aqueous solution, at a corresponding concentration of 5-10 wt%, and the adjusted pH is 6.5-7.5. Both the hydrochloric acid aqueous solution and the sulfuric acid aqueous solutions can be used in production to adjust the pH of an alkaline solution to be neutral or weakly acidic, with a wide range of applications and no chemical reaction with main products. A low concentration means a large amount, and a high concentration will generate a large amount of dissolution heat during configuration and is not conducive to the labor safety. If the final pH is too low, a large number of fatty acids will be attached to the main product oryzanol, which is not conducive to purification of oryzanol; and if the pH is too high, the main product cannot be completely precipitated, leading to low yield.

Preferably, at the step 5), the time for complete precipitation is 5-12h. If the precipitation time is short, the oryzanol will not be completely precipitated, thus reducing the yield; and if the precipitation time is long, the production cycle is affected, thus reducing the production efficiency.

Further preferably, at the step 5), the centrifugation manner is bag centrifugation. By means of comparing sedimentation centrifugation, bag centrifugation, horizontal spiral centrifugation and disc centrifugation, the inventor of the present invention finds that bag centrifugation has the best effect, and can obtain the centrifugated precipitates that is drier, lighter in colour and good in properties and has the dry humidity, purity and colour which cannot be achieved by other centrifugation manners.

At the step 6), the high-concentration ethanol is edible ethanol at a concentration greater than 85%, and the warm water refers to purified water with a temperature of 40-45°C, such as deionised water, distilled water, and ultrapure water. When the temperature exceeds 45°C, oryzanol absorbs water and swells significantly, and absorbs more impurities, and it is difficult to remove water from this part of swelling material, which is not conducive to the removal of residual ethanol; and when the temperature is less than 40°C, the effect of dissolving and removing ethanol is slightly poor. This temperature range is controlled to enhance the effect of removing ethanol and impurities.

By means of comparing various organic solvents, in combination with production practice, the present invention shows that ethyl acetate has significant advantages: It has low toxicity, and is basically non-toxic; it is not a solvent that is prohibited by Europe, America and Japan, that is, it can be used in production; it has little solubility for oryzanol: basically insoluble, and has excellent solubility for lipophilic impurities and pigments, so oryzanol can be refined. Ethyl acetate has dual advantages of purification and decolourisation. The high-concentration edible ethanol and ethyl acetate are mutually soluble, and the remaining ethyl acetate can be removed by centrifugation; purified water at a certain temperature and the edible ethanol are mutually soluble, and the remaining edible ethanol can be removed by centrifugation.

Preferably, at the step 6), in the washing step using the high-concentration ethanol and/or warm water, 50-70% high-concentration ethanol/or warm water is added into the centrifugated precipitates of the previous step, and is fully stirred, uniformly mixed and centrifugated; and the remaining high-concentration ethanol/or warm water is added into a centrifuge in batches. The number of additions in batches is not particularly limited. Generally, the remaining washing liquid can be uniformly added twice to four times.

Preferably, the mass of the centrifugated precipitates and the volume of the washing liquid, i.e., the ethyl acetate, the high-concentration ethanol and the warm water, at the step 6) are in accordance with a mass-volume ratio (kg/L) of 1-2:2-5:2-5:3-6, preferably 1-1.3:2-3:3-4:3.5-5. The washing order is in accordance with the above three solutions, and the centrifugation manner is bag centrifugation. That is, the ethyl acetate is used first, then the high-concentration edible ethanol, and the last the purified water at a certain temperature. In two adjacent solvents, the latter solvent can remove residues of the former solvent by means of bag centrifugation and washing with the washing liquid that is added in a small amount at each time. The present invention proves through experiments that the ethyl acetate is firstly used to purify oryzanol, the high-concentration edible ethanol is then used to dissolve out the ethyl acetate which is then removed by the bag centrifugation, and the edible ethanol is finally dissolved out by the purified water at a certain temperature and removed by the bag centrifugation. Through the combined use of the above three solvents, in combination with the bag centrifugation operation, the solvent residues are removed while refining the oryzanol, thus improving the product quality and safety.

At the step 7), the drying manner is not particularly limited, and examples that can be exemplified comprise blast drying, vacuum drying, or microwave vacuum drying, and the drying temperature is 55-65°C. The physicochemical properties of the oryzanol crystals are relatively stable at 55-65°C. One or a combination of the above drying manners can be selected to improve production efficiency.

The principle of the present invention is as follows: Oryzanol-containing soapstock, i.e., an oryzanol-containing by-product produced by alkali refining of animal and vegetable oils, is used as a raw material to extract oryzanol, and rice bran oil soapstock is preferred. The raw material mainly contains oryzanol, fatty acids, lipophilic impurities, fat-soluble pigments, etc., and these ingredients can be fully dissolved by edible ethanol at a concentration greater than 85% at a pH of 8.5-9.5 and a temperature of 50-60°C, and oryzanol is relatively stable under these conditions. Under the heat preservation condition of 50-60°C, a suitable filtration manner is selected to separate the impurities insoluble in the alkaline ethanol from the solution, and a clear and transparent solution can be obtained, thereby ensuring the normal progress of the refining operation and the clarity of the final product solution. According to the dissolution characteristics of the oryzanol and the impurities under this condition, their molecular weights and molecular structures, and the temperature of the system, a suitable ultrafiltration membrane is selected for membrane separation, so that during decolourisation, the purity of oryzanol is also improved. A molecular weight range of 1000-5000 is selected to obtain oryzanol with a light colour and high content; and the solution subjected to ultrafiltration membrane separation has a certain colour. According to the lipophilic characteristics of the oil, the inorganic decolourisers such as the activated clay and the activated carbon can be combined to further remove the fat-soluble impurities and pigments, and further purify the oryzanol, and the influence on the yield of oryzanol is minimised; and the remaining inorganic decolouriser powder in the solution can be completely removed by means of plate and frame filtration. After the aforementioned technological separation and purification, oryzanol and some impurities still exist in the solution. According to the nature that the oryzanol is insoluble in acidic ethanol, the oryzanol can be precipitated by adjusting the pH of the solution to be 6.5-7.5, and most of the impurities are dissolved in the acidic ethanol; and the oryzanol and the impurities are further separated by centrifugation to obtain purer oryzanol; and the oryzanol obtained after centrifugation still contains a few of lipophilic impurities and pigments. The present invention uses a specific order of washing, i.e., ethyl acetate, high-concentration ethanol, and warm water. The ethyl acetate has significant advantages: It has low toxicity, and is basically non-toxic; it is not a solvent that is prohibited by Europe, America and Japan, and can be used in production; it has little solubility for oryzanol, is basically insoluble, and has excellent solubility for lipophilic impurities and pigments, so oryzanol can be refined. The ethyl acetate has dual advantages of purification and decolourisation. The high-concentration edible ethanol and the ethyl acetate are mutually soluble, and the remaining ethyl acetate can be removed by centrifugation; purified water at a certain temperature and the edible ethanol are mutually soluble, and the remaining edible ethanol can be removed by centrifugation. Through the combined use of the above three solvents, in combination with the bag centrifugation operation, the solvent residues are removed while refining the oryzanol, thus improving the product quality and safety.

The present invention provides a white industrial product with an actual oryzanol content greater than 99%, that is, both the purity and the colour meet the standards. Furthermore, the solvents used in the product production process and the solvent residues meet the export standards.

The method of the present invention has the following beneficial effects:
I. The oryzanol obtained according to the method of the present invention is white powder. It is detected via ultraviolet-visible spectroscopic absorption chromatography (UV) that the obtained oryzanol is white high-quality oryzanol product having the purity up to 98% or above and containing a few of impurities.
II. The final yield of the oryzanol is greater than 73%. The oryzanol preparation method provided by the present invention has the characteristics of high purity and yield.
III. In the production process of extracting and preparing the oryzanol according to the present invention, the temperature is controlled at 50-60°C throughout the whole process. The inventor finds that the content and yield of the oryzanol in the product can achieve the best balance within this temperature range.
IV. The solvents used in the product production process and the solvent residues meet the export standards, and highly toxic solvents such as methanol, and extremely flammable and explosive organic solvents such as ether, hexane, and solvent oil are not used; and after testing, there is no solvent residues, thus improving the product use safety.
V. The present invention establishes an industrialised method suitable for extraction and preparation of oryzanol; the extraction process is simple, and requirements on production equipment are low; high-quality oryzanol industrial products can be produced on a large scale; and the method is low in production cost and is suitable for industrialised large-scale production.

### BRIEF DESCRIPTION OF THE DRAWINGS

No content.

### DETAILED DESCRIPTION

The method of an extraction process of the present invention will be further described below in combination with embodiments.

A raw material used in the embodiment of the present invention is oryzanol-containing soapstock which is purchased from Hunan Dongxiang Oil Co., Ltd., and the content of oryzanol is 13.65%. The purity of edible ethanol used in the embodiment of the present invention is 95%, and the edible ethanol is diluted with deionised water to a required concentration when needed; and the purity of ethyl acetate is 99.5% (food grade). Chemical reagents and raw and auxiliary materials used in the embodiment of the present invention are all obtained through conventional commercial channels, unless otherwise specified.

The content of oryzanol in a product is detected by ultraviolet-visible spectroscopic absorption chromatography (UV).

### Embodiment 1

1. Alkali-alcohol thermal dissolution: 2000 L of edible ethanol at a concentration of 89% is first added into a 6m³ extraction tank, and the tank is turned on for stirring; 600 kg of oryzanol-containing soapstock is fed into the extraction tank in constant stirring; and 2200 L of edible ethanol at the concentration of 89% is continued to be added. A cover is closed to continuously carry out stirring; cooling water is turned on, and steam is fed for heating. When the temperature is 54°C, 4wt% of a sodium hydroxide aqueous solution metered is added into the extraction tank to adjust the pH of the system to be 9.0. A sample is taken to observe dissolution of the solution; the raw material is completely dissolved after 20 min, and a few of oil residues remain; and 4500 L of solution is obtained.
2. Heat-preserved fine filtration: the temperature is maintained at 54°C; a precision filter with a folding filter element having a pore size of 0.45 um is configured; the solution is filtered by the precision filter; an operating pressure is maintained at 0.3 MPa, and a clear filtrate is obtained.
3. Heat-preserved ultrafiltration membrane separation: the temperature is maintained at 54°C; two groups of ultrafiltration membrane tubes are pre-assembled with high temperature resistant membranes (GE membranes of Duratherm HWS UF series) which are made of polyether sulfone and can withstand the highest temperature of 70°C, and respectively have molecular weight cut-off of 5000 (FLD-UF001) and 1000 (FLD-UF005); a membrane operating pressure is maintained at 1.5 MPa; the solution is firstly filtered with the ultrafiltration membrane with the molecular weight cut-off of 5000, and membrane downstream liquid is then collected; 100 L of edible ethanol at the concentration of 89% is added to clean membrane residues, and feeding permeated liquid and cleaning permeated liquid are combined to obtain permeated liquid; the permeated liquid is filtered with the ultrafiltration membrane with the molecular weight cut-off of 1000, and membrane upstream liquid is collected; 4550 L of cut-off liquid is obtained, i.e., membrane separation liquid.
4. Heat-preserved decolourisation: the temperature is maintained at 54°C; 32 kg of activated clay and 32 kg of activated carbon are added into the membrane separation liquid for full stirring for 20 min; a plate and a frame are matched with 1000-mesh canvas filter cloth for filtration to obtain light yellow clear and transparent decolourised liquid.
5. Acid neutralisation and filtration: under constant stirring, the pH of the decolourised liquid is adjusted to 7.0 with dilute sulphuric acid at a concentration of 6%, and stirring is stopped; running water is fed for cooling; standing is carried out for 8 h to cause precipitates to be completely precipitated; the precipitates are centrifugated through a three-foot cloth bag; centrifugated precipitates are collected; and 204 kg of wet centrifugated precipitates are obtained.
6. Washing: 500 L of ethyl acetate is added into the centrifugated precipitates for full stirring and uniform mixing; the mixture is centrifugated through the three-foot cloth bag, and then centrifugated precipitates are collected; 600 L of edible ethanol at the concentration of 89% is added into the precipitates for fully stirring and uniform mixing; the mixture is centrifugated through the three-foot cloth bag; the edible ethanol at the concentration of 89% is added into a centrifuge twice, 50 L at each time, for full permeation and precipitation, and centrifugated precipitates are collected; 600 L of purified water at a temperature of 40°C is added into the precipitates for full stirring and uniform mixing; the mixture is centrifugated through the three-foot cloth bag, and the purified water at the temperature of 40°C is added into the centrifuge for three times, 50 L at each time, for full permeation and precipitation; and centrifugated precipitates are collected.
7. Drying: the precipitates are vacuum-dried with microwaves, and the drying temperature is controlled at 62°C to obtain 63.26 kg of a finished oryzanol product.

After testing, the content of the finished oryzanol product is 99.12%, and the product yield is 76.56%.

### Embodiment 2

1. Alkali-alcohol thermal dissolution: 1000 L of edible ethanol at a concentration of 92% is first added into a 3m³ extraction tank, and the tank is turned on for stirring; 250 kg of oryzanol-containing soapstock is fed into the extraction tank in constant stirring; and 1000 L of edible ethanol at the concentration of 92% is continued to be added. A cover is closed to continuously carry out stirring; cooling water is turned on, and steam is fed for heating. When the temperature is 52°C, 6% of a sodium hydroxide aqueous solution metered is added into the extraction tank to adjust the pH of the system to be 9.3. A sample is taken to observe dissolution of the solution; the raw material is completely dissolved after 15min, and a few of oil residues remain; and 2100 L of solution is obtained.
2. Heat-preserved fine filtration: the temperature is maintained at 52°C; a precision filter with a folding filter element having a pore size of 0.45 um is configured; the solution is filtered by the precision filter; an operating pressure is maintained at 0.25 MPa, and a clear filtrate is obtained.
3. Heat-preserved ultrafiltration membrane separation: the temperature is maintained at 52°C; two groups of ultrafiltration membrane tubes are pre-assembled with imported high temperature resistant membranes which are made of polyether sulfone and can withstand the highest temperature of 70°C, and respectively have molecular weight cut-off of 5000 and 1000; a membrane operating pressure is maintained at 1.7 MPa; the solution is firstly filtered with the ultrafiltration membrane with the molecular weight cut-off of 5000, and membrane downstream liquid is then collected; 100 L of edible ethanol at the concentration of 92% is added to clean membrane residues, and feeding permeated liquid and cleaning permeated liquid are combined to obtain permeated liquid; the permeated liquid is filtered with the ultrafiltration membrane with the molecular weight cut-off of 1000, and membrane upstream liquid is collected; 2150 L of cut-off liquid is obtained, i.e., membrane separation liquid.
4. Heat-preserved decolourisation with an inorganic decolouriser: the temperature is maintained at 52°C; 20 kg of activated clay and 10 kg of activated carbon are added into the membrane separation liquid for full stirring for 20 min; a plate and a frame are matched with 1000-mesh canvas filter cloth for filtration to obtain light yellow clear and transparent decolourised liquid.
5. Acid neutralisation and filtration: under constant stirring, the pH of the decolourised liquid is adjusted to 7.2 with dilute hydrochloric acid at a concentration of 9%, and stirring is stopped; running water is fed for cooling; standing is carried out for 10 h to cause precipitates to be completely precipitated; the precipitates are centrifugated through a three-foot cloth bag; centrifugated precipitates are collected; and 83kg of wet centrifugated precipitates are obtained.
6. Washing: 250 L of ethyl acetate is added into the centrifugated precipitates for full stirring and uniform mixing; the mixture is centrifugated through the three-foot cloth bag, and then centrifugated precipitates are collected; 230 L of edible ethanol at the concentration of 92% is added into the precipitates for fully stirring and uniform mixing; the mixture is centrifugated through the three-foot cloth bag; the edible ethanol at the concentration of 92% is added into a centrifuge twice, 50 L at each time, for full permeation and precipitation, and centrifugated precipitates are collected; 250 L of purified water at a temperature of 40°C is added into the precipitates for full stirring and uniform mixing; the mixture is centrifugated through the three-foot cloth bag, and the purified water at the temperature of 40°C is added into the centrifuge for three times, 50 L at each time, for full permeation and precipitation; and centrifugated precipitates are collected.
7. Drying: the precipitates are dried with blast air, and the drying temperature is controlled at 65°C to obtain 25.70 kg of a finished oryzanol product which is white powder.

After testing, the content of the finished oryzanol product is 99.35%, and the product yield is 74.82%.

### Embodiment 3

Other steps are the same as those in Embodiment 1, and a difference is that the temperature of the system is controlled at 50°C throughout the whole process at the step 1) to the step 4). 60.81 kg of a finished oryzanol product is finally obtained. After testing, the content of the finished oryzanol product is 99.04%, and the product yield is 73.53%.

### Embodiment 4

Other steps are the same as those in Embodiment 1, and a difference is that the temperature of the system is controlled at 56°C throughout the whole process at the step 1) to the step 4). 62.11 kg of a finished oryzanol product is finally obtained. After testing, the content of the finished oryzanol product is 99.26%, and the product yield is 75.28%.

### Embodiment 5

Other steps are the same as those in Embodiment 1, and a difference is that the temperature of the system is controlled at 60°C throughout the whole process at the step 1) to the step 4). 61.53 kg of a finished oryzanol product is finally obtained. After testing, the content of the finished oryzanol product is 99.18%, and the product yield is 74.51%.

### Embodiment 6

Other steps are the same as those in Embodiment 1, and a difference is that the decolouriser at the step 4) only uses 64 kg of activated clay. 61.18 kg of a finished oryzanol product is finally obtained. After testing, the content of the finished oryzanol product is 98.67%, and the product yield is 73.71%.

### Embodiment 7

Other steps are the same as those in Embodiment 1, and a difference is that the decolouriser at the step 4) only uses 64 kg of activated carbon. 61.90 kg of a finished oryzanol product is finally obtained. After testing, the content of the finished oryzanol product is 98.25%, and the product yield is 74.26%.

### Embodiment 8

Other steps are the same as those in Embodiment 1, and a difference is that at the step 3), the solution is firstly filtered with the ultrafiltration membrane with the molecular weight cut-off of 1000, and then the membrane downstream liquid is collected; 100 L of edible ethanol at the concentration of 92% is added to clean membrane residues, and feeding permeated liquid and cleaning permeated liquid are combined to obtain permeated liquid; and the permeated liquid is then filtered with the ultrafiltration membrane with the molecular weight cut-off of 5000. 61.09 kg of a finished oryzanol product is finally obtained. After testing, the content of the finished oryzanol product is 98.14%, and the product yield is 73.20%.

### Comparative example 1

Other steps are the same as those in Embodiment 1, and a difference is that the temperature of the system is controlled at 46°C throughout the whole process at the step 1) to the step 4). 57.79kg of a finished oryzanol product is finally obtained. After testing, the content of the finished oryzanol product is 95.38%, and the product yield is 67.30%.

### Comparative example 2

Other steps are the same as those in Embodiment 1, and a difference is that the temperature of the system is controlled at 43°C throughout the whole process at the step 1) to the step 4). At the step 3), during heat-preserved ultrafiltration membrane separation, the membranes are blocked, and products cannot be effectively produced.

### Comparative example 3

Other steps are the same as those in Embodiment 1, and a difference is that the temperature of the system is controlled at 65°C throughout the whole process at the step 1) to the step 4). 54.46kg of a finished oryzanol product is finally obtained. After testing, the content of the finished oryzanol product is 96.72%, and the product yield is 64.31%.

### Comparative example 4

Other steps are the same as those in Embodiment 1, and a difference is that at the step 6) of washing, the ethyl acetate is replaced by isoamyl alcohol. 61.94 kg of a finished oryzanol product is finally obtained. After testing, the content of the finished oryzanol product is 95.84%, and the product yield is 72.48%.

### Comparative example 5

Other steps are the same as those in Embodiment 1, and a difference is that at the step 6) of washing, the washing order is changed: 500 L of ethyl acetate is added into the centrifugated precipitates for full stirring and uniform mixing; the mixture is centrifugated through the three-foot cloth bag, and then centrifugated precipitates are collected; 600 L of purified water at the temperature of 40°C is added into the precipitates for fully stirring and uniform mixing; the mixture is centrifugated through the three-foot cloth bag, and the purified water at the temperature of 40°C is added into a centrifuge for three times, 50 L at each time, for full permeation and precipitation; centrifugated precipitates are collected; 600 L of edible ethanol at the concentration of 89% is added into the precipitates for fully stirring and uniform mixing; the mixture is centrifugated through the three-foot cloth bag, and the edible ethanol at the concentration of 89% is added into the centrifuge for three times, 50 L at each time, for full permeation and precipitation; and centrifugated precipitates are collected. 61.75 kg of a finished oryzanol product is finally obtained. After testing, the content of the finished oryzanol product is 96.38%, and the product yield is 72.67%.

### Comparative example 6

Other steps are the same as those in Embodiment 1, and a difference is that at the step 6) of washing, washing with the ethyl acetate is omitted, and ethanol and warm water are directly used for washing. 64.75kg of a finished oryzanol product is finally obtained. After testing, the content of the finished oryzanol product is 91.36%, and the product yield is 72.23%.

## Claims

1. A method for preparing oryzanol from oryzanol-containing soapstock serving as a raw material, comprising the following steps:
1) alkali-alcohol thermal dissolution: adding ethanol into the raw material, heating to 50-60°C, adjusting the system to alkalescence, and dissolving the raw material to obtain a solution;
2) heat-preserved fine filtration: maintaining the temperature in the step 1), and using a precision filter to filter the solution;
3) heat-preserved ultrafiltration membrane separation: under a condition of 50-60°C, carrying out membrane separation on the filtrate by a high temperature resistant ultrafiltration membrane, and collecting membrane separation liquid in a molecular weight range of 1000-5000;
4) heat-preserved decolourisation: adding an inorganic decolouriser into the membrane separation liquid, and fully stirring and filtering the liquid to obtain decolourised liquid;
5) acid neutralisation and filtration: neutralising the decolourised liquid with dilute acid, cooling the liquid, standing the liquid to completely separate out precipitates, carrying out centrifugation, and collecting the centrifugated precipitates;
6) washing: adding ethyl acetate, ethanol at a concentration greater than 85% and purified water with a temperature of 40-45°C in sequence into the centrifugated precipitates for washing, wherein the washing is to add a washing liquid of the above solvent or solution into the centrifugated precipitates, stir and mix the product thoroughly, and centrifugate the mixture to obtain centrifugated precipitates for next step of washing; and
7) drying: drying the precipitates to obtain a finished oryzanol product,
wherein in the step 1) to the step 4) the system is maintained at a temperature of 50-60°C; and wherein in the step 4), the decolouriser comprises activated clay and activated carbon at a mass ratio of 1-2:1-2, and the adding amount of the decolouriser is 3-6% by weight of the oryzanol-containing soapstock raw material.

2. The method according to claim 1, **characterized in that** at the step 1), the ethanol refers to ethanol at a concentration greater than 80%; the adding amount of the ethanol is 5-10 times the weight of an oryzanol-containing soap base; the adjustment to alkalescence refers to use of an alkaline hydroxide, and the hydroxide to adjust the pH to 8.5-9.5.

3. The method according to claim 1, **characterized in that** at the step 2), for filtration, a precision filter with a folding filter element is used; the filter element has a pore size of 0.45 µm and an operating pressure of 0.2-0.4 MPa.

4. The method according to claim 1, **characterized in that** at the step 3), the high temperature resistant ultrafiltration membrane is made of polyether sulfone, and is capable of withstanding a temperature of 80°C.

5. The method according to claim 1, **characterized in that** at the step 3), the molecular weight cut-off of the membrane is set to be 1000-5000, and a membrane operating pressure is 1.0-2.0 MPa.

6. The method according to claim 5, **characterized in that** the order of the membrane separation is: firstly, the solution is filtered with an ultrafiltration membrane with a molecular weight cut-off of 5000; membrane downstream liquid, i.e., permeated liquid, is then collected; the permeated liquid is filtered with an ultrafiltration membrane with a molecular weight cut-off of 1000; and membrane upstream liquid, i.e., cut-off liquid, is collected.

7. The method according to claim 1, **characterized in that** a mass-volume ratio (kg/L) of the mass of the centrifugated precipitates to the volume of the washing liquid, the high-concentration ethanol and the warm water, at the step 6) is in the scope of 1-2:2-5:2-5:3-6.

## Patentansprüche

1. Verfahren zur Herstellung von Oryzanol aus oryzanolhaltigem Seifenstock, der als Rohmaterial dient, umfassend die folgenden Schritte:
1) thermische Auflösung mit Alkalialkohol: zugabe von Ethanol zum Rohmaterial, erhitzen auf 50-60°C, einstellen des Systems auf Alkaleszenz und Auflösen des Rohmaterials, um eine Lösung zu erhalten;
2) wärmekonservierte Feinfiltration: Aufrechterhalten der Temperatur in Schritt 1) und Verwendung eines Präzisionsfilters zum Filtern der Lösung;
3) wärmekonservierte Ultrafiltrationsmembrantrennung: unter einer Bedingung von 50-60°C, Durchführen einer Membrantrennung des Filtrats durch eine hochtemperaturbeständige Ultrafiltrationsmembran und Sammeln von Membrantrennflüssigkeit in einem Molekulargewichtsbereich von 1000-5000;
4) wärmekonservierte Entfärbung: zugabe eines anorganischen Entfärbungsmittels zur Membrantrennflüssigkeit, vollständiges Rühren und Filtern der Flüssigkeit, um eine entfärbte Flüssigkeit zu erhalten;
5) Säureneutralisation und Filtration: neutralisieren der entfärbten Flüssigkeit mit verdünnter Säure, abkühlen der Flüssigkeit, stehenlassen der Flüssigkeit zur vollständigen Abtrennung der Niederschläge, durchführen einer Zentrifugation und Sammeln der zentrifugierten Niederschläge;
6) Waschen: zugabe von Ethylacetat, Ethanol mit einer Konzentration von mehr als 85% und gereinigtem Wasser mit einer Temperatur von 40-45° C nacheinander zu den zentrifugierten Niederschlägen zum Waschen, wobei das Waschen darin besteht, den zentrifugierten Niederschlägen eine Waschflüssigkeit des oben genannten Lösungsmittels oder der oben genannten Lösung zuzusetzen, das Produkt gründlich zu rühren und zu mischen und die Mischung zu zentrifugieren, um zentrifugierte Niederschläge für den nächsten Waschschritt zu erhalten; und
7) Trocknen: trocknen der Niederschläge, um ein fertiges Oryzanol-Produkt zu erhalten, wobei von Schritt 1) bis zu Schritt 4) das System auf einer Temperatur von 50-60°C gehalten wird; und wobei in Schritt 4) der Entfärber aktivierten Ton und Aktivkohle in einem Massenverhältnis von 1-2:1-2 umfasst und die Zugabemenge des Entfärbers 3-6 Gew.-% des oryzanolhaltigen Seifenstock-Rohmaterials beträgt.

2. Verfahren nach Anspruch 1, **dadurch gekennzeichnet, dass** in Schritt 1), das Ethanol sich auf Ethanol mit einer Konzentration von mehr als 80% bezieht; die Zugabemenge des Ethanols das 5-10-fache des Gewichts einer oryzanolhaltigen Seifenbasis beträgt; die Einstellung auf Alkaleszenz sich auf die Verwendung eines alkalischen Hydroxids bezieht, und das Hydroxids den pH-Werts auf 8,5-9,5 einstellt.

3. Verfahren nach Anspruch 1, **dadurch gekennzeichnet, dass** in Schritt 2) zur Filtration ein Präzisionsfilter mit einem Faltfilterelement verwendet wird; das Filterelement hat eine Porengröße von 0,45 µm und einen Betriebsdruck von 0,2-0,4 MPa.

4. Verfahren nach Anspruch 1, **dadurch gekennzeichnet, dass** in Schritt 3) die hochtemperaturbeständige Ultrafiltrationsmembran aus Polyethersulfon besteht und einer Temperatur von 80 °C standhalten kann.

5. Verfahren nach Anspruch 1, **dadurch gekennzeichnet, dass** in Schritt 3) die Molekulargewichtsgrenze der Membran auf 1000-5000 eingestellt wird und der Betriebsdruck der Membran 1,0-2,0 MPa beträgt.

6. Verfahren nach Anspruch 5, **dadurch gekennzeichnet, dass** die Reihenfolge der Membrantrennung folgendermaßen ist: zunächst wird die Lösung mit einer Ultrafiltrationsmembran mit einer Molekulargewichtsgrenze von 5000 gefiltert; die membranabwärts gelegene Flüssigkeit, d. h. die durchgedrungene Flüssigkeit, wird dann gesammelt; die durchgedrungene Flüssigkeit wird mit einer Ultrafiltrationsmembran mit einer Molekulargewichtsgrenze von 1000 gefiltert; und die membranaufwärts gelegene Flüssigkeit, d. h. die Grenzflüssigkeit, wird gesammelt.

7. Verfahren nach Anspruch 1, **dadurch gekennzeichnet, dass** ein Masse-VolumenVerhältnis (kg/L) der Masse der zentrifugierten Niederschläge zum Volumen der Waschflüssigkeit, des hochkonzentrierten Ethanols und des warmen Wassers in Schritt 6) im Bereich von 1-2:2-5:2-5:3-6 liegt.

## Revendications

1. Procédé de préparation d'oryzanol à partir d'un stock de savon contenant de l'oryzanol servant de matière première, comprenant les étapes suivantes:
1) dissolution thermique avec alcali-alcool: ajouter de l'éthanol dans la matière première, chauffer à 50-60°C, ajuster le système à l'alcalescence et dissoudre la matière première pour obtenir une solution;
2) filtration fine à température maintenue: maintenir la température à l'étape 1), et utiliser un filtre de précision pour filtrer la solution;
3) séparation par membrane d'ultrafiltration à température maintenue: dans des conditions de 50 à 60°C, effectuer une séparation par membrane sur le filtrat par une membrane d'ultrafiltration résistante aux hautes températures, et recueillir le liquide de séparation par membrane dans une plage de poids moléculaire de 1 000 à 5 000;
4) décoloration thermo-conservée: ajouter un décolorant inorganique dans le liquide de séparation par membrane, et agiter complètement et filtrer le liquide pour obtenir un liquide décoloré;
5) neutralisation avec acid et filtration: neutraliser le liquide décoloré avec de l'acide dilué, refroidir le liquide, laisser reposer le liquide pour séparer complètement les précipités, effectuer une centrifugation et recueillir les précipités centrifugés;
6) lavage: ajouter de l'acétate d'éthyle, de l'éthanol à une concentration supérieure à 85% et de l'eau purifiée à une température de 40-45°C en séquence dans les précipités centrifugés pour le lavage, dans lequel le lavage consiste à ajouter un liquide de lavage du solvant ou de la solution ci-dessus dans les précipités centrifugés, agiter et mélanger complètement le produit, et centrifuger le mélange pour obtenir des précipités centrifugés pour la prochaine étape de lavage; et
7) séchage: sécher les précipités pour obtenir un produit fini d'oryzanol, dans lequel, de l'étape 1) à l'étape 4), le système est maintenu à une température de 50 à 60°C; et dans lequel à l'étape 4), le décolorant comprend de l'argile activée et du charbon activé dans un rapport massique de 1 -2:1-2, et la quantité ajoutée du décolorant est de 3 à 6% en poids de la matière première de stock de savon contenant de l'oryzanol.

2. Procédé selon la revendication 1, **caractérisé en ce qu'**à l'étape 1), l'éthanol désigne l'éthanol à une concentration supérieure à 80%; la quantité ajoutée d'éthanol est de 5 à 10 fois le poids d'un stock de savon contenant de l'oryzanol; l'ajustement à l'alcalescence fait référence à l'utilisation d'un hydroxyde alcalin, et l'hydroxyde pour ajuster le pH à 8,5-9,5.

3. Procédé selon la revendication 1, **caractérisé en ce qu'**à l'étape 2), pour la filtration, un filtre de précision avec un élément filtrant repliable est utilisé; l'élément filtrant a une taille de pores de 0,45 µm et une pression de fonctionnement de 0,2-0,4 MPa.

4. Procédé selon la revendication 1, **caractérisé en ce qu'**à l'étape 3), la membrane d'ultrafiltration résistante aux hautes températures est en polyéther sulfone, et est capable de résister à une température de 80°C.

5. Procédé selon la revendication 1, **caractérisé en ce qu'**à l'étape 3), le seuil de coupure poids moléculaire de la membrane est fixé à 1000-5000, et une pression de fonctionnement de la membrane est de 1,0-2,0 MPa.

6. Procédé selon la revendication 5, **caractérisé en ce que** l'ordre de la séparation par membrane est: dans un premier temps, la solution est filtrée avec une membrane d'ultrafiltration avec un seuil de coupure poids moléculaire de 5000; le liquide en aval de la membrane, c'est-à-dire le liquide ayant subi une perméation, est ensuite collecté; le liquide ayant subi une perméation est filtré avec une membrane d'ultrafiltration avec un seuil de coupure poids moléculaire 1000; et le liquide en amont de la membrane, c'est-à-dire le liquide de coupure, est collecté.

7. Procédé selon la revendication 1, **caractérisé en ce qu'**un rapport masse-volume (kg/L) de la masse des précipités centrifugés au volume du liquide de lavage, de l'éthanol à haute concentration et de l'eau chaude, à l'étape 6) est de l'ordre de 1-2:2-5:2-5:3-6.
